# EUROPEAN PATENT APPLICATION

(11) **EP 4 524 153 A1**
(43) Date of publication of application: **19.03.2025**
(21) Application number: 23802792.4
(22) Date of filing: 06.05.2023
(51) Int. Cl.: C07K 16/00, C07K 16/42, A61K 39/395, A61K 45/00, A61P 35/00, A61P 29/00, A61P 37/02, A61P 1/00, A61P 13/12, A61P 25/28, A61P 25/16, A61P 19/02

(54) **Vk4-1-IGLC POLYPEPTIDE AND USE THEREOF**

(30) Priority: 07.05.2022 CN 202210492107
(71) Applicant: Guangzhou Sig Biopharmaceutical Technology Co., Ltd., Guangzhou, Guangdong 510555 (CN)
(72) Inventor: QIU, Xiaoyan, Beijing 100085 (CN); WANG, Qianqian, Beijing 100085 (CN); JIANG, Wenhua, Beijing 100085 (CN); HE, Zhiqiao, Beijing 100085 (CN)
(74) Representative: Flesselles, Bruno F.G.
(86) International application number: PCT/CN2023/092458
(87) International publication number: WO 2023/217020

(57) **Abstract**

Disclosed in the present invention are an immunoglobulin x-type light chain (Vκ4-1-IgLC) polypeptide having a unique Vκ4-1 and the use thereof. The amino acid sequence of the V4-1-IgLC polypeptide is as shown in SEQ ID NO. 1, and the polypeptide can be used as a target or a marker for preparing a drug for diagnosing and/or treating tumors or inflammatory diseases, and can also be used for preparing an Integrin-FAK signal pathway inhibitor. An antibody prepared by using the polypeptide as an antigen can effectively inhibit the development of tumors, and provides a new thought for clinical diagnosis and treatment of tumors.

## Description

### FIELD OF THE INVENTION

The present invention relates to the technical field of immunity and the diagnosis and treatment of cancer, and specifically relates to an immunoglobulin x-type light chain (Vκ4-1-IgLC) polypeptide having a unique Vκ4-1 and the use thereof.

### BACKGROUND OF THE INVENTION

According to classical immunological theory, the basic structure of immunoglobulin (Ig) consists of four peptide chains, that is, two identical heavy chains and two identical light chains are connected by disulfide bonds to form a complete Ig molecule. In addition to Ig molecules with the classical tetrapeptide chain structure, some light chains that do not bind to Ig heavy chains are commonly present in normal human peripheral blood and a variety of body fluids. These light chains are called free light chains (FLCs), which exist in the form of monomers (molecular weight 22~27 kDa), in the form of covalently or non-covalently bound dimers (44-55 kDa), or in the form of polymers, such as the Bence-Jones protein (BJP) in urine.

Traditionally, FLC has been considered as an excess byproduct produced by B cells, which has no function under physiological conditions. However, there is growing evidence showing that FLC is significantly associated with the progression and severity of inflammatory diseases such as autoimmune diseases, diabetes and inflammation of the central nervous system. In particular, two refractory diseases, amyloid protein immunoglobulin light chain (AL) and light chain deposition disease (LCDD), have been identified as being mediated by abnormally folded insoluble FLCs. Both AL and LCDD are refractory diseases caused by irreversible deposition of free κ (kappa) or λ (lambda) chains in the extracellular space, characterized by misfolding of free light chains and deposition in tissues and organs, often in the heart, kidney, liver and lung, resulting in tissue structure damage, organ dysfunction and progressive progression. AL-associated FLCs show a unique filamentous structure in tissues, and LCDD-associated FLCs show amorphous deposition in tissues. Both AL and LCDD exhibit common monoclonal features, but lack diversity. In addition, the κ chains of AL- or LCDD-associated FLCs typically exhibit the same Vκ4-1/Jκ3 rearrangement. To date, AL- and LCDD-associated FLCs have been considered as being associated with B-cell malformations. However, an increasing number of cases have shown the absence of abnormal proliferation of B cells or plasma cells, suggesting that there may be other sources of the FLC.

There are two types of immunoglobulin light chains, κ and λ, and the κ chains are often used more frequently during the process of encoding Ig in B lymphocytes. Also, same as the heavy chains, the κ chains also have diversity in Ig molecules, as there are 40 V fragments in the κ chain gene, and these fragments can be randomly recombined with 5 J fragments to form a complete coding sequence of the κ chain variable region, so the κ chains expressed by different B lymphocytes present different combinations of Vκ and VJ, and similarly, the hypervariable regions (the CDR3 regions, which predominantly determine the specificity of the antibody) encoded by some Vκ fragments and some VJ fragments are also different. However, our study results (CN1940069A) found that different types of tumor cells have identical or very similar sequences of the light chain variable region, that is, presenting a specific Vκ4-1/J3 combination sequenc. Also, the CDR3 sequence in the Vκ4-1/J3 combination is highly conserved in the Ig light chain variable region derived from tumor cells, and it is specific compared with the CDR3 derived from B lymphocytes, which shows the difference between tumor cells and B lymphocytes. Studies have shown that its CDR3 sequence can be used as a target for diagnosing or treating tumors.

However, in subsequent studies, we made new discoveries and thus obtained the present invention.

### SUMMARY OF THE INVENTION

Our studies have found that immunoglobulin x-type light chains with unique Vκ4-1 are widely expressed in cancer cells of different lineages and expressed at a low level in non-cancer cells. In addition, we obtained the sequence of a fragment of the free light chain Vκ4-1, which can be used to label free Ig light chains having Vκ4-1. On this basis, the technical solution of the present invention is described in detail as follows:
In a first aspect, the present invention provides a Vκ4-1-IgLC polypeptide with an amino acid sequence as shown in 5'-TQSPDSLVVSLGERASINCKSQRVSLG-3' (SEQ ID NO: 1).

In a second aspect, the present invention provides use of the above-mentioned polypeptide in preparing a drug for diagnosing and/or treating malignant tumors and/or inflammation.

Alternatively or preferably, in the above-mentioned use, the malignant tumors include at least one of the following: glioma, medulloblastoma, large cell lung cancer, esophageal cancer, gastric cancer, colorectal cancer, breast cancer, renal cell carcinoma, prostate cancer, liver cancer, pancreatic cancer, skin cancer, oral cancer, seminoma, osteosarcoma, leiomyosarcoma, angiosarcoma, liposarcoma, synovial sarcoma, rhabdomyosarcoma, B-cell lymphoma, T-cell lymphoma, leukemia and myeloma.

Alternatively or preferably, in the above-mentioned use, the inflammatory diseases include at least one of the following: systemic lupus erythematosus, rheumatoid arthritis, multiple sclerosis, inflammatory bowel disease, inflammatory nephropathy, systemic amyloidosis, Alzheimer's disease (presenile dementia) and Parkinson's disease.

In a third aspect, the present invention provides an antibody drug, which is prepared by using the above-mentioned polypeptide as an antigen, and the antibody drug is a recognizing antibody or a blocking antibody.

Alternatively or preferably, the above-mentioned antibody drug is a monoclonal antibody, and the amino acid sequence of the heavy chain variable region thereof is as shown in SEQ ID NO: 3 or SEQ ID NO: 4.

Alternatively or preferably, the amino acid sequence of the light chain variable region of the above-mentioned antibody drug is as shown in SEQ ID NO: 5.

In a fourth aspect, the present invention also provides a small molecule drug, which is prepared by using the gene sequence or mRNA sequence of the above-mentioned polypeptide as a target. The small molecule drug includes, but is not limited to, gRNA and siRNA, and can specifically target the gene sequence or mRNA sequence of the above-mentioned polypeptide, thereby blocking its expression.

In a fifth aspect, the present invention also provides use of the above-mentioned polypeptide in preparing an Integrin-FAK signaling pathway inhibitor.

The present invention has the following beneficial effects:
Based on the sequence analysis of an immunoglobulin x-type light chain having a unique Vκ4-1, the present invention obtains a functional polypeptide, which is located in the conserved region of Vκ4-1 and can specifically label a free Ig light chain sequence containing a unique Vκ4-1. Antibodies prepared based on this polypeptide, or polypeptides or small molecules that can specifically bind to this target can specifically recognize and bind to the immunoglobulin κ-type light chain with Vκ4-1 in malignant tumors and/or inflammatory disease tissues, and inhibit its function, thereby significantly inhibiting the growth of cancer cells or inhibiting inflammation *in vitro* and *in vivo.* Further molecular studies have found that the antibodies based on this polypeptide can also inhibit the activation of the Integrin-FAK signaling pathway, and can be used as Integrin-FAK signaling pathway inhibitors.

### DESCRIPTION OF THE DRAWINGS

Figure 1 shows the complete amino acid sequence of the immunoglobulin κ-type light chain having a unique Vκ4-1 and the functional annotation of each moiety.
Figure 2 shows that the monoclonal antibody of Example 2 specifically recognizes Vκ4-1/Jκ3-FLC in the cell lysate, but does not recognize Vκ1-5/Jκ3-LC. Lane 1 and Lane 2 are 293T cell lysates transfected with Vκ1-5/Jκ3-LC and Vx4-1/Jx3-FLC plasmids fused with Myc tag, respectively, and an anti-Myc tag antibody is as control.
Figure 3 shows the sequence alignment of the heavy chain variable regions of the monoclonal antibodies 6G5 (Ighv2-3) and 5D3 (Ighv2-9) of Example 2.
Figure 4 shows the immunohistochemistry result of Vκ4-1/Jκ-FLC in four cancer tissues as detected by 5D3 in Example 3.
Figure 5 shows the statistics of the immunohistochemistry score of Vκ4-1/Jκ-FLC in more types of cancer tissues as detected by 6G5 in Example 3.
Figure 6 shows the immunohistochemistry result of Vκ4-1/Jκ-FLC in tissues from different parts of patients with colorectal cancer as detected by 6G5 and 5D3 in Example 3. The left panel shows the immunohistochemistry photos of two cases, the upper right shows the histological score of all cases as detected by 6G5, and the lower right shows the histological score of all cases as detected by 5D3.
Figure 7 is a figure of the immunofluorescence staining result of different cancer cell lines stained with 5D3 in Example 4.
Figure 8 is a figure of the Western blot result of a colorectal cancer cell line as detected by 6G5 in Example 4.
Figure 9 shows the identification of Vκ4-1/Jκ-FLC recombinant protein obtained by the CHO expression system in Example 5, wherein (A) the Vx4-1/Jx-FLC recombinant protein secreted by CHO cells into the supernatant was detected on Day 6 of transfection, (B) Vκ4-1/Jκ-FLC was purified from the culture supernatant, (C) the purified Vκ4-1/Jκ-FLC recombinant protein was detected by Western blot and PAGE, and (D) the Vκ4-1/Jκ-FLC recombinant protein was analyzed by using Superdex200 molecular sieve column.
Figure 10 shows the experimental result of r-Vκ4-1/Jκ-FLC promoting tumor cell proliferation in Example 5. *, P<0.05; ***, P<0.001.
Figure 11 shows the experimental result of r-Vκ4-1/Jκ-FLC promoting tumor cell migration and invasion in Example 5. **, P<0.01; ***, P<0.001; ****, P<0.0001. Bar, 200 µm.
Figure 12 shows the result of cell migration ability of HT-29 cells and SW480 cells after knocking out Igκ as detected by Transwell assay in Example 5. A represents SW480 cells, and B represents HT-29 cells. **, P<0.01; ***, P<0.001; ****, P<0.0001. Bar, 200 µm.

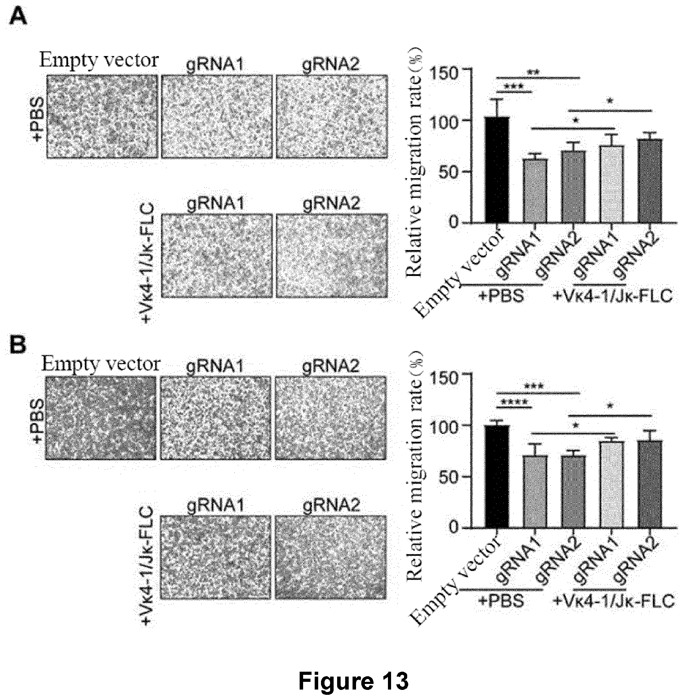

Figure 13 shows the result that the migration inhibition caused by knocking down Igκ can partially be rescued by addition of exogenous recombinant protein r-Vκκ4-1/Jκ-FLC, as detected by Transwell assay in Example 5. A is the result of cell migration ability of Igκ-knockdown SW480 after addition of 1 µg/ml of r-Vκ4-1/Jκ-FLC to the culture system, as detected by Transwell assay; B is the result of cell migration ability of Igκ-knockdown HT-29 after addition of 1 µg/ml of r-Vκ4-1/Jκ-FLC to the culture system, as detected by Transwell assay. *, P<0.05; **, P<0.01; ***, P<0.001; ****, P<0.0001; bar, 200 µm.
Figure 14 shows the experimental result that cell migration inhibition by Vκ4-1/Jκ-FLC can be blocked by the monoclonal antibody 5D3 in Example 5. A is for SW480 cells, B is for HT-29 cells, ns represents not significant; *, P<0.05; ****, P<0.0001. Bar, 200 µm.
Figure 15 shows the result of tumor cell migration ability after addition of exogenous 5D3 and exogenous 5D3+r-Vκ4-1/Jκ-FLC, as detected by Transwell in Example 5. A is for SW480 cells, B is for HT-29 cells, ns represents not significant; *, P<0.05; **, P<0.01; ****, P<0.0001. Bar, 200 µm.
Figure 16 shows a tumor photo of the tumor model established with SW480 cells after injection of r-Vκ4-1/Jκ-FLC in Example 5, as well as the statistical results of tumor volume and weight. *, P<0.05; **, P<0.01.
Figure 17 shows a tumor photo of the CDX model after treatment with 6G5 in Example 6, as well as the statistical results of the tumor volume and weight after *in vivo* detection and experiment. ***, P<0.001, ****, P<0.0001.
Figure 18 shows a tumor photo of the PDX model after treatment with 6G5 in Example 6, as well as the statistical results of the tumor volume and weight after *in vivo* detection and experiment. ***, P<0.001, ****, P<0.0001.
Figure 19 shows a tumor photo of the CDX model after treatment with 5D3 in Example 6, as well as the statistical results of the tumor volume and weight after *in vivo* detection and experiment. ***, P<0.001, ****, P<0.0001.
Figure 20 shows a tumor photo of the PDX model after treatment with 5D3 in Example 6, as well as the statistical results of the tumor volume and weight after *in vivo* detection and experiment. ***, P<0.001, ****, P<0.0001.
Figure 21 shows the Western blot detection result of the major molecules of the Integrin-FAK signaling pathway after addition of the monoclonal antibody to the culture of the tumor cells in Example 7. A is for SW480 cells, and B is for HT-29 cells.
Figure 22 shows the Western blot detection result of the major molecules of the Integrin-FAK signaling pathway after intervention of the two tumor models with the monoclonal antibody 6G5 in Example 7. A is for the CDX model, and B is for the PDX model.
Figure 23 shows the Western blot detection result of the major molecules of the Integrin-FAK signaling pathway after intervention of the two tumor models with the monoclonal antibody 5D3 in Example 7. A is for the CDX model, and B is for the PDX model.
Figure 24 shows the effect of the monoclonal antibody 5D3 on the growth of osteosarcoma in nude mice in Example 8. The upper panel shows the change of tumor volume in mice of the 5D3 group and the mIgG control group as a function of treatment days, and the lower panel shows the comparison of the tumor sizes between the two groups of mice.
Figure 25 shows the electrophoresis result of the free Vκ4-1/Jκ light chain in the serum of MS patients and healthy humans as detected by using an antibody that specifically recognizes the 28-amino-acid peptide in Example 9.
Figure 26 shows the staining result of the brain tissues of AD patients and normal humans as detected by 6G5 in Example 10, wherein A shows dendritic strong staining in AD patients, B shows focal and plaque staining in AD patients, C shows diffusive precipitation staining in AD patients, and D is for normal control.
Figure 27 shows the SDS-PAGE electrophoresis result of the serum of cardiac amyloidosis patients and normal humans as detected by using 6G5 in Example 11.
Figure 28 shows the detection of Vκ₄₋₁-type free Ig light chain polypeptide in the glomerular basement membrane of patients with focal nephropathy as detected by 6G5 and mouse IgG in Example 12. A shows the fluorescent staining result for 6G5, and B shows the fluorescent staining result for mouse IgG.
Figure 29 shows the staining result of the pathological sections of different inflammatory gastrointestinal diseases as detected by using 5D3 in Example 13, wherein A is for inflammatory colon polyps, B is for ulcerative colitis, C is for chronic gastritis, and D is for normal colon tissue.

### DETAILED DESCRIPTION OF THE INVENTION

The technical solution of the present invention is explained in detail below with reference to the preferred examples, so that those skilled in the art can better understand and implement the technical solutions.

### Example 1. Discovery of a light chain with unique Vκ4-1 rearrangement pattern

Our previous studies on tumor epithelial cells such as breast cancer, colorectal cancer and squamous cell lung cancer showed that all variable regions of the Igκ light chain had Vx4-1, and these patterns has been disclosed (see GenBank: AY505537-AY505541). In addition, we found that Vκ4-1 was highly homologous to hydrophobic FLC, and Vκ4-1 was found in LCDD or AL.

Subsequently, we selected tissue samples from 5 cases of colorectal cancer, including cancerous tissue, paracancerous tissue and distal normal tissue. Samples were obtained from Peking University People's Hospital with written informed consent. The study was conducted in accordance with the protocol approved by the Institutional Review Board and was approved by the Clinical Research Ethics Committee of Peking University People's Hospital (2015PHB212-01). The obtained cancer tissues were sorted for EpCAM⁺ cancer cells by flow cytometry, and the sorting method was as follows:
First, the tissue was cut into small pieces (about 1 mm³) and washed with 1× PBS. Epithelial cells were isolated from the tissue by incubating at 37°C for 1 hour and shaking in 1× PBS containing 5 mmol/L EDTA and 5 mmol/L DTT. The digested epithelial cells were detached from a gentleMACS Dissociator (Miltenyi Biotec) and filtered through a nylon mesh. Cells were then washed with 1× PBS containing 2% fetal bovine serum (FBS) (10099141, Gibco) for 3 times, blocked in 1× PBS containing 5% fetal bovine serum at 4°C for 30 minutes, and stained with anti-human CD19 (11-0199-41, eBioscience) and anti-human EpCAM (12-9326-42, eBioscience) at 4°C for 30 minutes. EpCAM⁺ cells were then subjected to fluorescence-activated cell sorting (FACS) by FACSAria II (BD Biosciences) to obtain EpCAM⁺ cancer cells.

Next, EpCAM⁺ cancer cells were used as the research object to study the transcripts of Igκ light chain variable region by multiplex RT-PCR amplification and IR-Seq sequencing technology.

Sample preparation and sequencing for IR-Seq: total RNA was extracted from the sorted EpCAM⁺ cancer cells by Trizol Reagent (15596018, Life Technology). Two rounds of PCR were performed by using the iRepertoire^{®} commercial kit with the Igκ (iRepertoire Inc.) primer set under the reaction conditions specified in the instructions of the kit. In the first round, reverse transcription was completed, and a tag and sequencing primers were introduced into the PCR product using nested gene-specific primers that were complementary to the V and C genes. The second round of PCR used common (sequencing) primers for exponential amplification. The DNA concentration of the eluted PCR product was detected, and 100 ng of DNA was collected for sequencing. Subsequent quality control and sequencing were conducted by Novogene.

A predominance of light chain with a unique Vκ4-1 pattern was found in 5 patients.

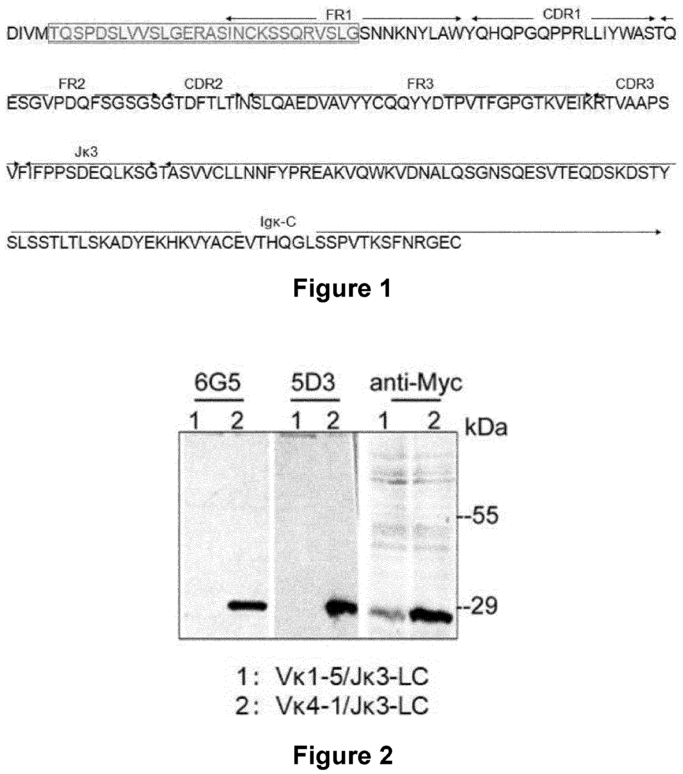

According to the IR-Seq sequencing results, the full-length sequence of the Igκ chain with a unique Vκ4-1 variable region was obtained as follows:
DIVMTQSPDSLVVSLGERASINCKSSORVSLGSNNKNYLAWYQHQPGQPPR LLIYWASTQESGVPDQFSGSGSGTDFTLTINSLQAEDVAVYYCQQYYDTPVTFGP GTKVEIKRTVAAPSVFIFPPSDEQLKSGTASVVCLLNNFYPREAKVQWKVDNAL QSGNSQESVTEQDSKDSTYSLSSTLTLSKADYEKHKVYACEVTHQGLSSPVTKS FNRGEC (SEQ ID NO: 2), which was an immunoglobulin κ-type light chain with a unique Vκ4-1, and annotation of each functional region thereof was as shown in Figure 1.

Wherein, the 28 amino acids at positions 5-32 (SEQ ID NO: 1, all within the Vκ4-1 region) have function as specific target and can label the free immunoglobulin κ chains having a unique Vκ4-1 variable region.

### Example 2. Preparation of antibodies targeting the Ig light chain having a unique Vκ4-1 pattern

In order to understand the function of these non-B cell-derived Igκ light chain variable regions having a Vκ4-1 pattern, we prepared monoclonal antibodies by using the sequence as shown in SEQ ID NO: 1 (artificially synthesized) as an antigen.

Preparation of the monoclonal antibody:
6-8-week-old BALB/c female mice (purchased from Beijing Vital River Laboratory Animal Technology Co., Ltd.) were immunized with the above-mentioned 28-amino-acid polypeptide antigen conjugated to human albumin. Spleen cells were harvested after immunization, fused with myeloma cells, and screened for positive clones of hybridoma cell lines by using the above-mentioned 28-amino-acid polypeptide. The positive clones of hybridoma cells obtained after final screening were named 5D3 and 6G5, respectively.

10-week-old female BALB/c multiparous mice were intraperitoneally injected with incomplete Freund's adjuvant at 500 µL/animal, and hybridoma cells could be intraperitoneally injected one week later. 5D3 and 6G5 hybridoma cells were separately cultured in RPMI 1640 + 10% FBS + 1% PS medium at 37°C in a 5% CO₂ incubator. The expression of antibodies in the culture supernatant was detected. After expression was confirmed, hybridoma cells cultured to logarithmic growth phase were collected and centrifuged, and the supernatant was discarded. The cells were resuspended in pre-warmed serum-free 1640 medium and washed twice. The cells were aliquoted at 2.0×10⁶ hybridoma cells/500 µL for intraperitoneal injection for each mouse. The mice were separately subjected to intraperitoneal injection. When the mice showed significant production of ascitic fluid (about 7~10 days later), the ascitic fluid was collected with 9 gauge syringe needles under sterile conditions, and centrifuged at 4°C, 1200 rpm for 10 min to remove the hybridoma cells therein. 30% glycerol was added to the ascitic fluid supernatant and mixed well, and then the solution was aliquoted and stored at -20°C for later use.

The chromatography column was filled with an appropriate amount of protein G column packing, and washed and equilibrated with cold PBS (pH 7.4) at 10~20 times of column volume. The ascitic fluid with monoclonal antibody was centrifuged at 4°C, 10,000 rpm for 5 min, and tangible impurities were removed. The fluid was diluted with PBS at a ratio of 3:1, incubated with protein G column packing, and rotated at 4°C overnight. The next day, the fluid that passed through the column was collected and loaded to the column repeatedly for 10~20 times, and then allowed to flow through naturally. Then the column was washed with cold PBS (pH 7.4) at 10~20 times of column volume to remove non-specifically bound protein impurities. Then the bound antibody was eluted with 0.1 mol/L glycine-HCl buffer (pH 3.0), and immediately neutralized to pH 7.4 with 1 mol/L Tris-HCl (pH 11.0). After elution, the purification column was washed with PBS (pH 7.4) at 10~20 times of column volume and stored in 20% ethanol. The eluted antibody was ultrafiltered by PBS and quantified, added with 30%~50% glycerol, and stored at -20°C for later use. The whole operation procedure was carried out in a cold room at 4°C or on ice. The purity of antibody was detected by SDS-PAGE. The resulting antibodies corresponding to their hybridoma cell lines were named 5D3 and 6G5, respectively.

Evaluation of monoclonal antibody specificity:
Two 293T cell lysates respectively transfected with Vκ1-5/Jκ3-LC and Vκ4-1/Jκ3-LC plasmids (both with Myc tag) were used as experimental objects. The cell lysates after SDS-PAGE separation were incubated with 5D3 and 6G5, respectively, and an anti-Myc tag antibody was used as control, so as to detect the ability of the monoclonal antibodies for recognizing Vκ1-5 pattern and Vx4-1 pattern (LC: light chain).

The results were as shown in Figure 2. The electrophoresis results showed that both 6G5 and 5D3 specifically recognized Vκ4-1/Jκ3-LC, but not Vκ1-5/Jκ3-LC, indicating that they can specifically recognize the amino acid sequence at positions 5-32 of Vκ4-1. In the figure, anti-Myc represented anti-Myc tag antibody.

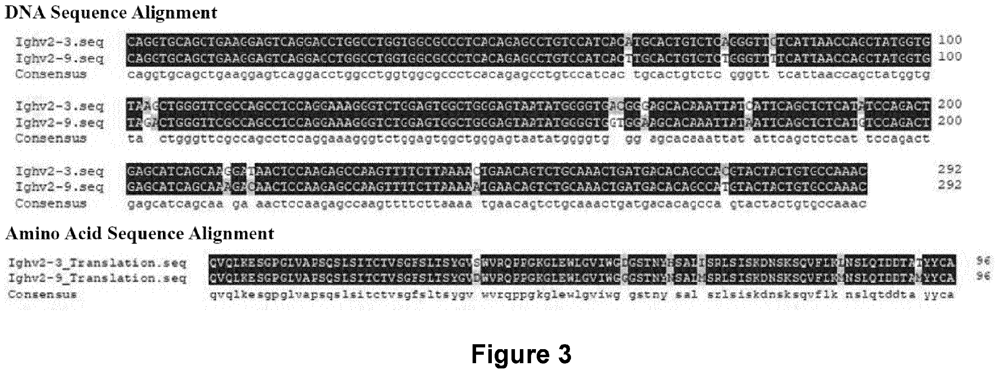

The samples were subjected to sequencing, and for the monoclonal antibody 5D3, the amino acid sequence of heavy chain variable region was as shown in SEQ ID NO: 4, the encoding gene was as shown in SEQ ID NO: 7, and the amino acid sequence of the light chain variable region was as shown in SEQ ID NO: 5, the encoding gene was as shown in SEQ ID NO: 8. For the monoclonal antibody 6G5, the amino acid sequence of heavy chain variable region was as shown in SEQ ID NO: 3, and the encoding gene was as shown in SEQ ID NO: 6. The alignment of the DNA sequences and amino acid sequences of the heavy chain variable regions between the two monoclonal antibodies was as shown in Figure 3.

### Example 3. Detection of immunoglobulin κ-type light chain having a unique Vκ4-1 in different cancer samples by monoclonal antibody

The light chain Igκ having a unique Vκ4-1 pattern, labeled as Vκ4-1/Jκ-Igκ, was detected by immunohistochemistry in tissue samples of endometrial cancer, colorectal cancer (colon cancer), breast cancer and esophageal cancer by using the monoclonal antibody 5D3, and normal tissue was used as a negative control.

Scoring criteria for immunohistochemistry: the scoring of cytoplasmic staining utilized four intensity grades (0, none; 1, weak; 2, medium; 3. strong) and the percentage of positive cells (0%-100%). The final score was the product of the intensity grade and the percentage of positive cells (range: 0-300). A final score of >100 was considered a strong positive stain, and a final score of ≤100 was considered a weak positive stain.

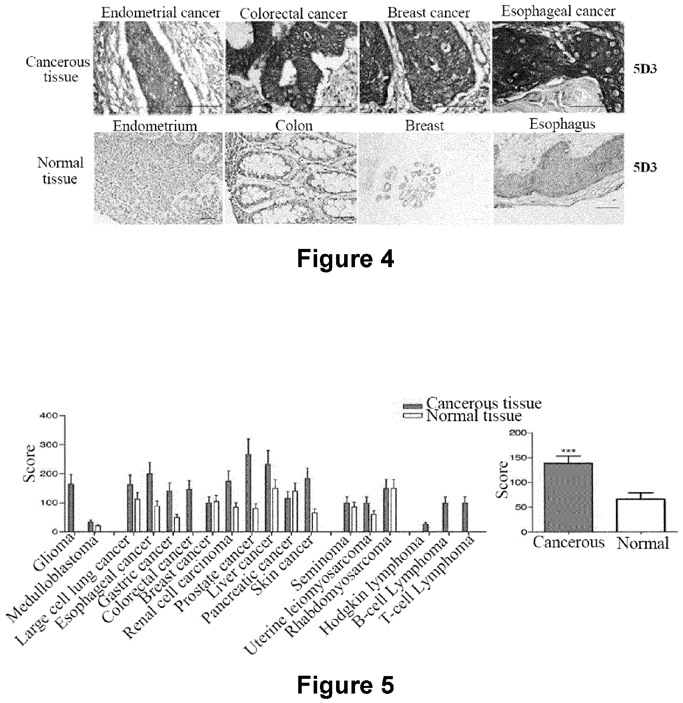

The results were as shown in Figure 4. The immunoglobulin κ-type light chain (immunoglobulin κ chain) having a unique Vκ4-1 was highly expressed in all four cancer tissues and lowly expressed in non-malignant-tumor cells (the staining of normal tissue in the normal group was very weak).

In addition, we selected more tumor tissues, including glioma, medulloblastoma, large cell lung cancer, esophageal cancer, gastric cancer, colorectal cancer (colon cancer), breast cancer, renal cell carcinoma, prostate cancer, liver cancer, pancreatic carcinoma, skin cancer, seminoma, osteosarcoma, uterine lelomyosarcoma (lelomyosarcoma), rhabdomyosarcoma, liposarcoma, angiosarcoma, synovialsarcoma, hodgkin lymphoma, B-cell lymphoma (B lymphoma) and T-cell lymphoma (T lymphoma), and subjected them to immunohistochemistry detection for Vκ4-1/Jκ-Igκ by using the monoclonal antibody 6G5, with normal tissue as a control.

The statistics of the detection results were as shown in Figure 5. The Vκ4-1/Jκ-Igκ recognized by the monoclonal antibody 6G5 was highly expressed in a variety of malignant tumor cells, and lowly expressed in non-malignant-tumor cells (normal tissues).

In order to further verify whether Vκ4-1/Jκ-Igκ was overexpressed in cancer cells, we compared the expression frequencies of Vκ4-1/Jκ-Igκ between cancer cells and normal epithelial cells in the same individual with colorectal cancer.

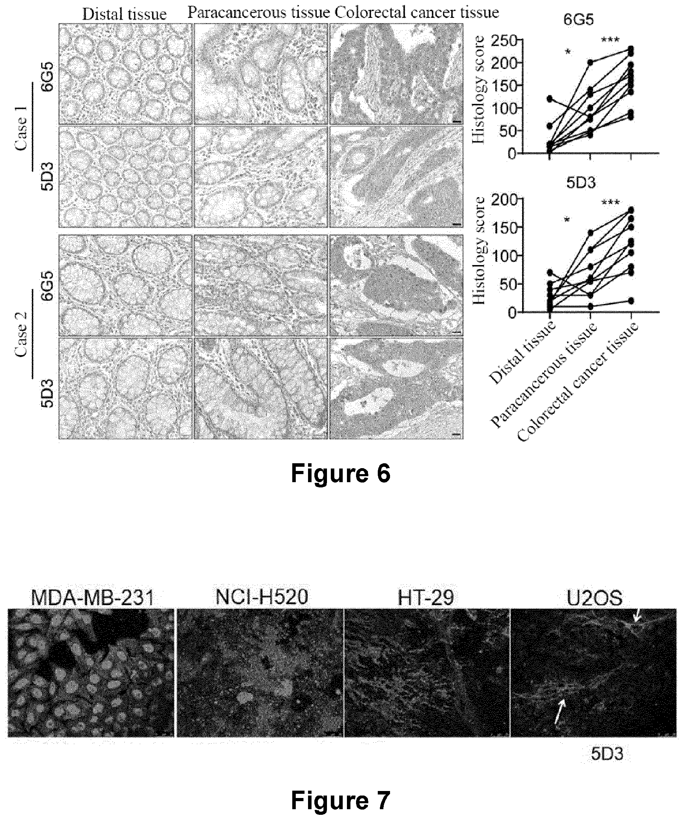

Tissues were collected from 10 patients with colorectal cancer. The tissue at the operative incision (distal tissue, which was normal tissue), paracancerous tissue and cancerous tissue were collected from each patient, and subjected to immunohistochemical staining by using 6G5 and 5D3, respectively. The results were as shown in Figure 6, which showed the results of two cases. No positive staining was seen in normal tissues, weak positive was seen in paracancerous tissues, and strong staining in cancerous tissues, and the results were consistent for both monoclonal antibodies. The results showed that the expression frequency of Vκ4-1/Jκ-Igκ in colorectal cancer tissues was significantly higher than that in normal tissues, and there was also a significant difference in the expression frequency of Vκ4-1/Jκ-Igκ between the colorectal cancer tissues and the paracancerous tissues. These results indicated that Vκ4-1/Jκ-Igκ was only widely expressed in cancer cells.

### Example 4. Study of the properties of Vκ4-1/Jκ-Igκ

We used the monoclonal antibody 5D3 as a detection tool and the cell lines MDA-MB-231 (breast cancer cell), NCI-H520 (squamous cell lung carcinoma cell), HT-29 (colorectal cancer cell), U2OS (osteosarcoma cell) (obtained from the American Type Culture Collection (ATCC) and maintained by the Peking University Center for Human Disease Genomics) as experimental objects to perform cell localization analysis of Vκ4-1/Jκ-Igκ by immunofluorescence, and to detect whether Vκ4-1/Jκ-Igκ was hydrophobic and free in both cancer cell lines.

Immunofluorescence: cell lines were quickly washed with PBS and fixed in 4% paraformaldehyde at room temperature for 15 minutes. The fixed cells were blocked with 5% BSA and perforated in the cell membrane (to enable the entry of antibodies into cells) with 0.02% Triton X-100 at room temperature for 30 minutes. Cells were then immunostained with 5D3 (10 µg/mL) diluted in blocking buffer at room temperature for 3 hours, and washed with PBS for three times for 5 minutes each time. Cells were incubated with Alexa594 anti-mouse secondary antibody and Alexa488 anti-rabbit secondary antibody (molecular probe) at room temperature for 1 hour and then washed. Images were captured by a confocal microscope using a Leica STED imager (Leica Microsystems).

Immunofluorescence was as shown in Figure 7, and the results showed that Vκ4-1/Jκ-Igκ could be secreted and deposited into the extracellular matrix.

Next, we used SW480 and HT-29 cell lines (both were colon cancer cell lines) as research subjects for Western blot detection. Samples for the four lanes of reducing electrophoresis were culture supernatant, negative control (medium control), cell lysate, and ECM extract, respectively. Samples for non-reducing electrophoresis were serum, SW480 cells, and HT-29 cells, respectively. Antibodies used in non-reducing electrophoresis were anti-human IgG antibody (anti IgG Fc), anti-human free Igκ antibody (anti kappa free light chain), and 6G5. 6G5 was used for reducing electrophoresis.

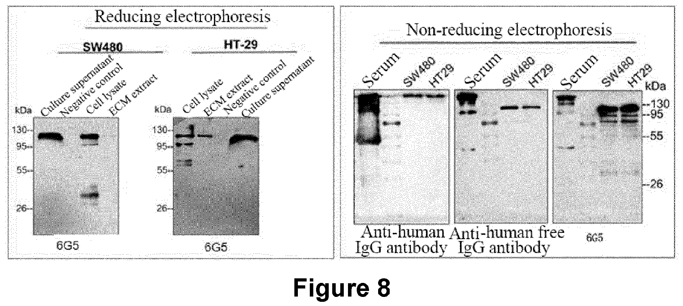

The results were as shown in Figure 8. The left panel was the result of reducing electrophoresis and the right panel was the result of non-reducing electrophoresis. The Igκ light chain having a Vx4-1/Jx sequence was mainly present in the cytoplasm, culture supernatant, and extracellular matrix as free multimer. Both reducing (left) and non-reducing (right) electrophoresis showed that the Ig light chain having a Vκ4-1/Jκ sequence was in a free form and was mainly present in the form of multimer. The Igκ light chain having a Vκ4-1/Jκ sequence did not form a 4-peptide chain structure with IgG heavy chain as detected by using anti-human IgG antibody (anti IgG Fc) under non-reducing electrophoresis conditions, while the Igκ light chain having a Vκ4-1/Jκ sequence was in a free form as detected by using anti-human free Igκ antibody (anti kappa free light chain), consistent with the signal of 6G5.

These results indicated that Vκ4-1/Jκ-Igκ showed a property of being free in both reduced and non-reduced states, and had a variety of forms, including monomer, dimer and polymer in culture supernatant and ECM extract. This suggested that non-B-cell-derived Vκ4-1/Jκ may be an ECM protein. The light chain Igκ having a unique Vκ4-1 in tissues, i.e., Vκ4-1/Jκ (Vκ4-1 rearranged in combination with other Jκ genes), was in fact a type of free Ig light chain, and was designated as Vκ4-1/Jκ-FLC hereinafter.

### Example 5. Promotion of tumor invasion and metastasis by Vκ4-1/Jκ-FLC

In order to explore the effect of Vκ4-1/Jκ-FLC on cancer development and progression, we prepared a biologically active recombinant protein r-Vκ4-1/Jκ-FLC using Chinese hamster ovary cells (CHO) as an eukaryotic expression platform.

The full-length gene sequence containing Vκ4-1 (SEQ ID NO: 2, with C and V regions) was cloned into a pGEX-4T-2 vector and further subcloned into a pcDNA3.1 myc-His(-)B vector by using the same restriction enzyme cleavage sites. The recombinant protein r-Vκ4-1/Jκ-FLC containing a His-tag at the C-terminus was expressed in CHO cells and purified by Capto^{™} L affinity chromatography (GE Healthcare, United States).

Purification and identification of the eukaryotic recombinant protein (CHO expression system): the sample was centrifuged at 3000 rpm, 4°C for 15 min, and the culture supernatant was collected. Capto L can specifically bind to the variable region of κ-type light chain and can be used to purify the Igκ light chain eukaryotic recombinant protein. The chromatography column was packed with an appropriate amount of Capto L and pre-equilibrated with cold equilibration solution (20 mmol/L sodium dihydrogen phosphate, 150 mmol/L NaCl, pH 7.2) at 10 times of column volume. The culture supernatant (input) was added to the column, which was sealed and subjected to rotary incubation at 4°C overnight. Then the liquid in the column was allowed to flow through the column (flow through), which was washed with cold equilibration solution to remove unbound and non-specifically bound proteins, and then eluted with cold elution solution (0.1 mol/L sodium citrate, pH 2.0-3.5). The eluent was neutralized to pH 7.4 with neutralization solution (1 mmol/L Tris-HCl, pH 11.0), concentrated by ultrafiltration, quantified by BCA and stored at -80°C for later use. The purified protein was identified by SDS-PAGE and Western blot.

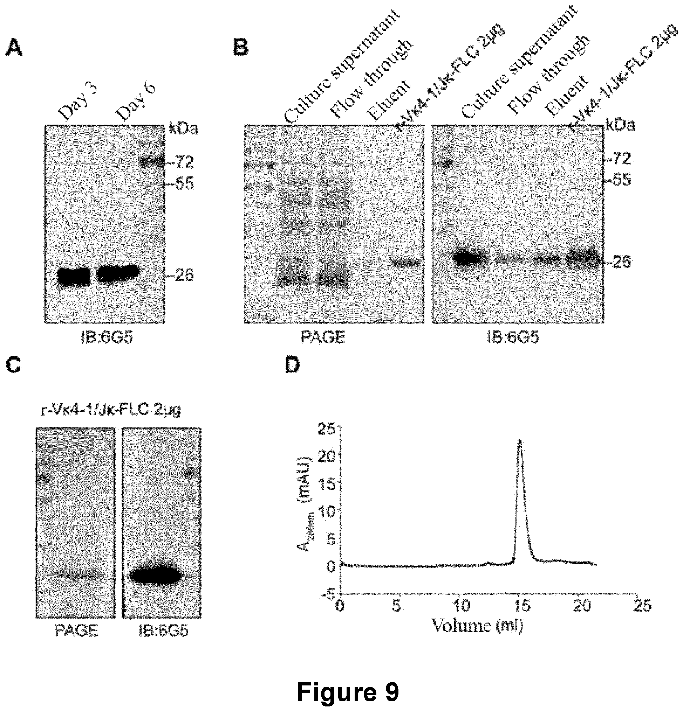

After successfully establishing the culture and expression system of the cell, we found that r-Vκ4-1/Jκ-FLC expressed by CHO cells could be secreted into the cell culture supernatant (Figure 9 A). We subjected the collected culture supernatant to affinity chromatography by using a CaptoL affinity column (specifically bound to the variable region of Ig κ-type light chain), and validated the purified recombinant protein by using SDS-PAGE, Western blot and Superdex200 column, confirming that we obtained the recombinant protein r-Vκ4-1/Jκ-FLC with high purity (Figure 9B-D).

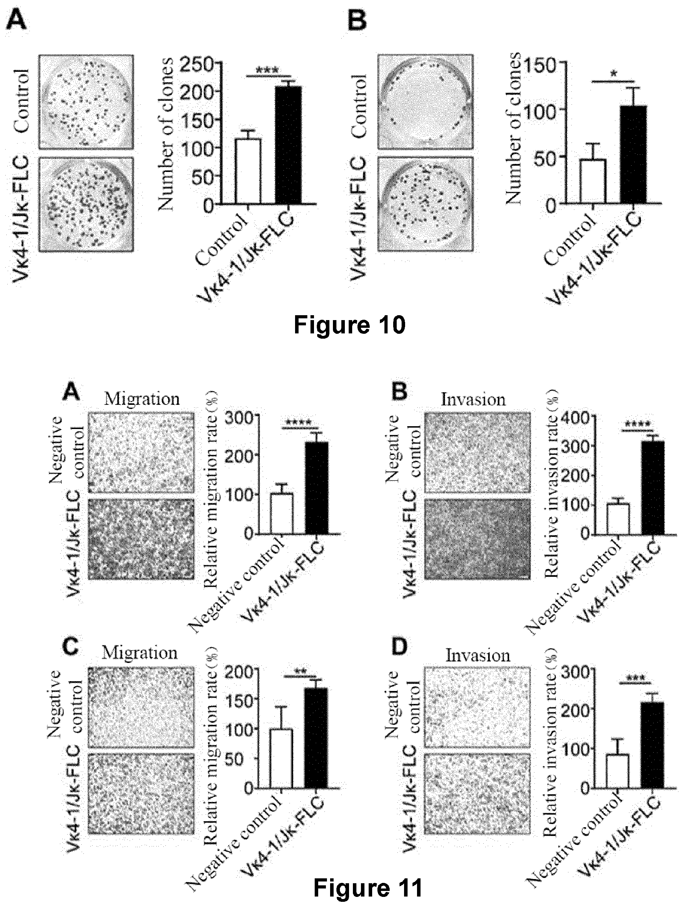

SW480 and HT-29 cells were used as the research objects, and r-Vκ4-1/Jκ-FLC was added for intervention culture. The experimental results were as shown in Figures 10 and 11, wherein in Figure 10, A showed the ability of cell proliferation and self-renewal of SW480 cells treated with 1 µg/mL r-Vκ4-1/Jκ-FLC as detected by clone formation assay. B showed the ability of cell proliferation and self-renewal of HT-29 cells treated with 1 µg/mL r-Vκ4-1/Jκ-FLC as detected by clone formation assay. In Figure 11, A and B showed the effects of r-Vκ4-1/Jκ-FLC on the ability of migration and invasion of SW480 cells, as detected by treating SW480 cells with 1 µg/mL r-Vκ4-1/Jκ-FLC and by Transwell assay (A) and Matrigel-Transwell assay (B), respectively. C and D showed the effects of r-Vκ4-1/Jκ-FLC on the ability of migration and invasion of SW480 cells, as detected by treating HT-29 cells with 1 µg/mL r-Vκ4-1/Jκ-FLC and by Transwell assay (C) and Matrigel-Transwell assay (D), respectively. The results showed that treatment with r-Vκ4-1/Jκ-FLC increased the ability of survival/proliferation and migration of SW480 and HT-29 cells compared with the negative control.

Next, we used three gRNAs targeting the constant region of Igκ to knock out Igκ in SW480 and HT-29 cells.

| Name of Igκ guide RNA | Sequence |
|---|---|
| gRNA-1 | F primer 5'-CACCGGGTGGATAACGCCCTCCAAT-3' |
| | R primer 5'-AAACATTGGAGGGCGTTATCCACCC-3' |
| gRNA-2 | F primer 5'-CACCGCCTGGGAGTTACCCGATTGG-3' |
| | R primer 5'- AAACCCAATCGGGTAACTCCCAGGC-3' |
| gRNA-3 | F primer 5'-CACCGTCTCCTGGGAGTTACCCGAT-3' |
| | R primer 5'-AAACATCGGGTAACTCCCAGGAGAC-3' |

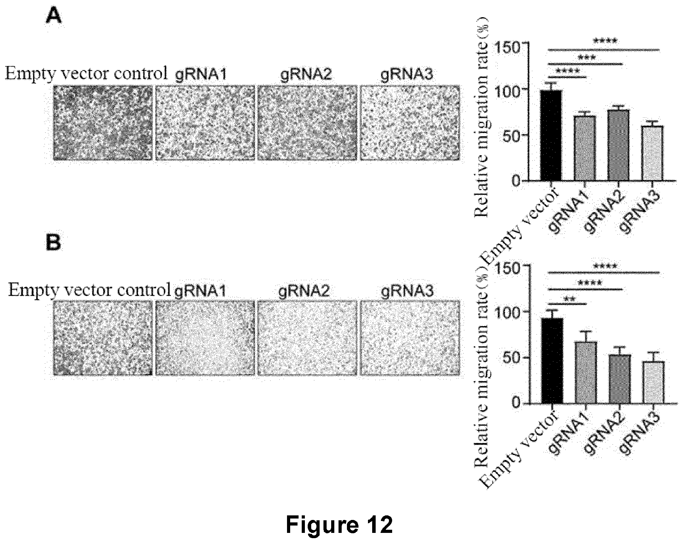

The results showed that the migration ability of both cells decreased after knocking out Ig. As can be seen from Figure 12, A showed the cell migration ability as detected by Transwell assay after knocking out Igκ in SW480 cells, and B showed the cell migration ability as detected by Transwell assay after knocking out Igκ in HT-29 cells. The cell migration ability was significantly reduced after knocking out Igκ by the three gRNAs compared with the control group of empty vector (vector).

In order to further demonstrate that Vκ4-1/Jκ-FLC exerted its pro-tumor effect primarily through the secreted form rather than intracellular Igκ, we first knocked out Igκ in cells and then added the recombinant protein r-Vκ4-1/Jκ-FLC to the culture system. The results of the Transwell assay showed that the exogenous addition of the recombinant protein r-Vκ4-1/Jκ-FLC could partially reverse the inhibition on the ability of cell migration due to Igκ knockout (Figure 13). These results demonstrated that Vκ4-1/Jκ-FLC mainly exerted pro-tumor effect in secreted form.

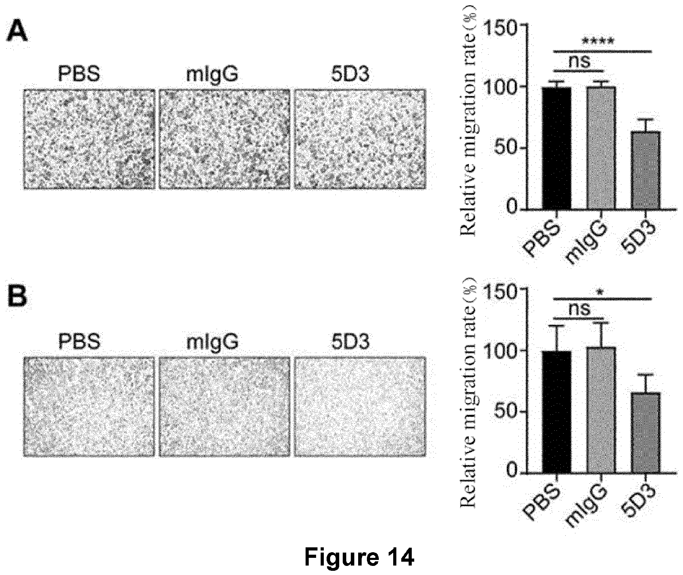

Further, in order to demonstrate that Vκ4-1/Jκ-FLC exerted pro-tumor effect primarily through the secreted form, we also added the 5D3 specific antibody to the culture system to block the secretory Vκ4-1/Jκ-FLC (Igκ secreted by the cells themselves). The results showed that the addition of 5D3 to block the secreted Vκ4-1/Jκ-FLC could significantly inhibit cell migration. The results were as shown in Figure 14. A showed the experimental result of cell migration ability as detected by adding 50 µg/mL 5D3 to SW480 cell culture supernatant and by Transwell, and B showed the experimental result of cell migration ability as detected by adding 50 µg/mL 5D3 to HT-29 cell culture supernatant and by Transwell. The results showed that the addition of 5D3 significantly reduced the migration ability of both cells compared with the negative controls PBS and mIgG.

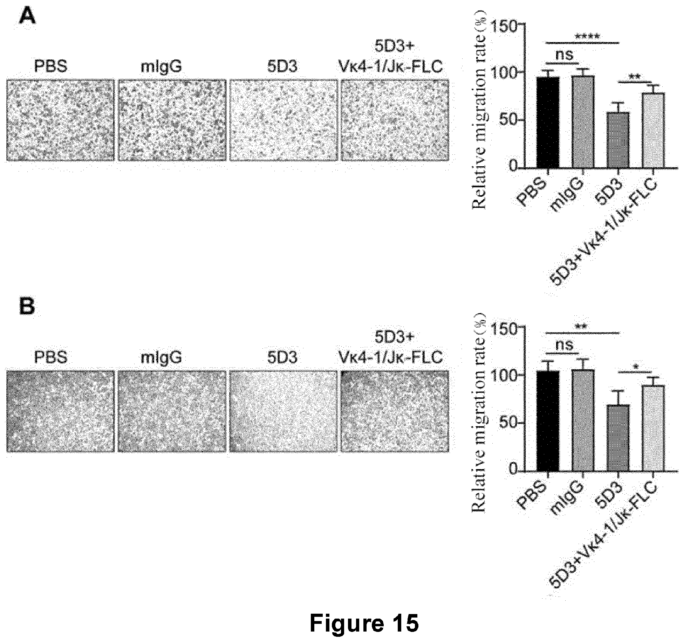

Subsequently, we added 50 µg/mL 5D3 to the SW480 and HT-29 cell culture supernatants, respectively, and simultaneously added 10 µg/mL r-Vκ4-1/Jκ-FLC to the culture systems, respectively, so as to detect the cell migration ability by Transwell assay. The experimental results were as shown in Figure 15. The addition of the exogenous recombinant protein r-Vκ4-1/Jκ-FLC could reverse the inhibitory effect of 5D3 antibody on cell migration after blocking by the antibody.

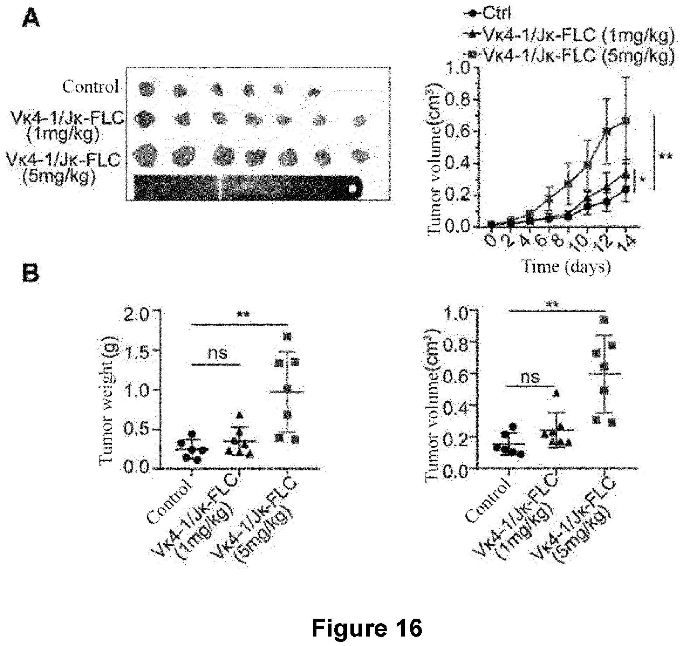

Further, we used SW480 cells to establish a subcutaneous xenograft tumor model in nude mice, and injected the recombinant protein r-Vκ4-1/Jκ-FLC peritumorally at different doses to detect the ability of tumor growth promotion of r-Vκ4-1/Jκ-FLC. The results showed that the recombinant protein r-Vκ4-1/Jκ-FLC promoted tumor growth in a dose-dependent manner, and both the tumor volume and weight were higher than those of the control group. As can be seen from Figure 16, A was the pictures of the three groups of tumors at the end of the experiment and the growth curves of tumor volumes; B was the statistical charts of the tumor volume and tumor weight of the three groups at the end of the experiment.

In conclusion, we confirmed that the Vκ4-1/Jκ-FLC secreted by tumor cells played an important role in the proliferation and metastasis of colorectal cancer by using *in vivo* and *in vitro* experiments.

### Example 6. Inhibition of tumor growth by monoclonal antibodies 5D3 and 6G5

Given that Example 5 has verified that the monoclonal antibody 5D3 could specifically block the pro-tumor properties of Vκ4-1/Jκ-FLC *in vitro,* we next examined the effect of the monoclonal antibodies 6G5 and 5D3 on the growth of colon cancer xenografts in SCID mice.

The animal models were classified into two groups, one group of model was established by subcutaneously injecting SW480 cells to SCID mice (CDX), and the other group of model was established by subcutaneously injecting tumor cells from colon cancer patients to SCID mice (PDX). After tumor formation, intravenous administration (5 mg/kg) was given every 4 days, and mouse IgG (mIgG) was used as a control to dynamically observe the tumor growth rate and to dynamically monitor the change in mouse body weight. At the end of the experiment, the differences in tumor volume and tumor weight between 6G5 and the control group, and the difference in tumor volume and tumor weight between 5D3 and the control group were compared.

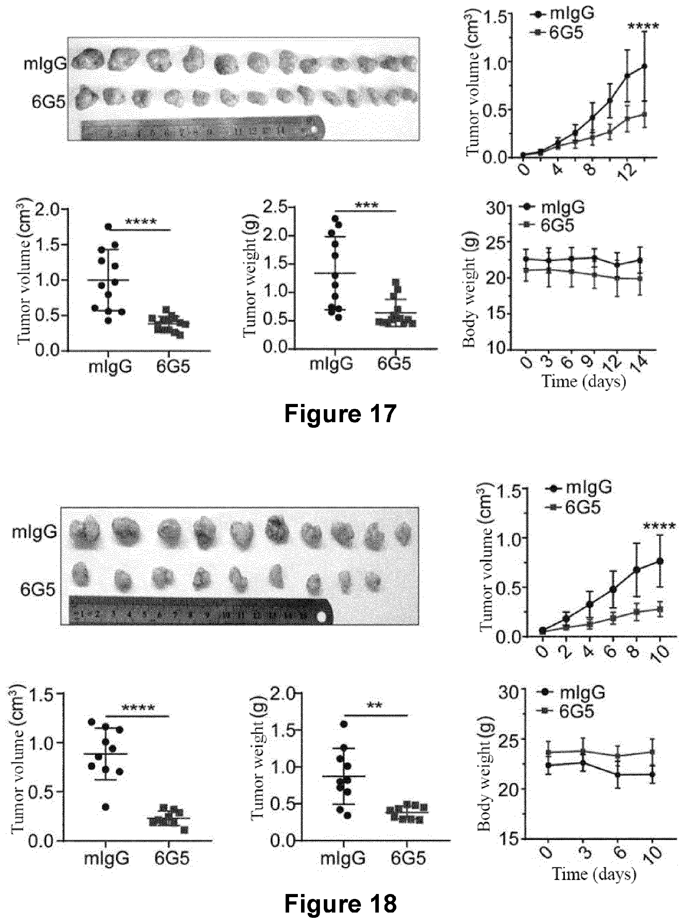

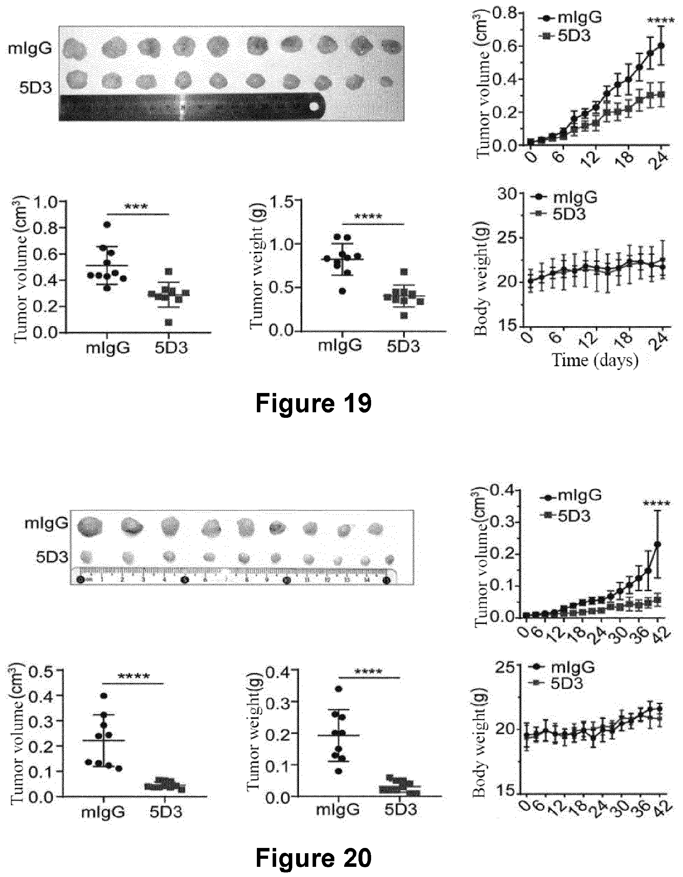

The experimental results of the two groups were as shown in Figures 17~18 (6G5) and Figures 19~20 (5D3).

The results showed that both the monoclonal antibodies 6G5 and 5D3 had anti-tumor effect, and significantly inhibited the increase of tumor volume and weight compared with the control group, but had no significant effect on the body weight of mice, which was comparable to that of the control group.

### Example 7. Inhibition of the Integrin-FAK signaling pathway by monoclonal antibodies 5D3 and 6G5

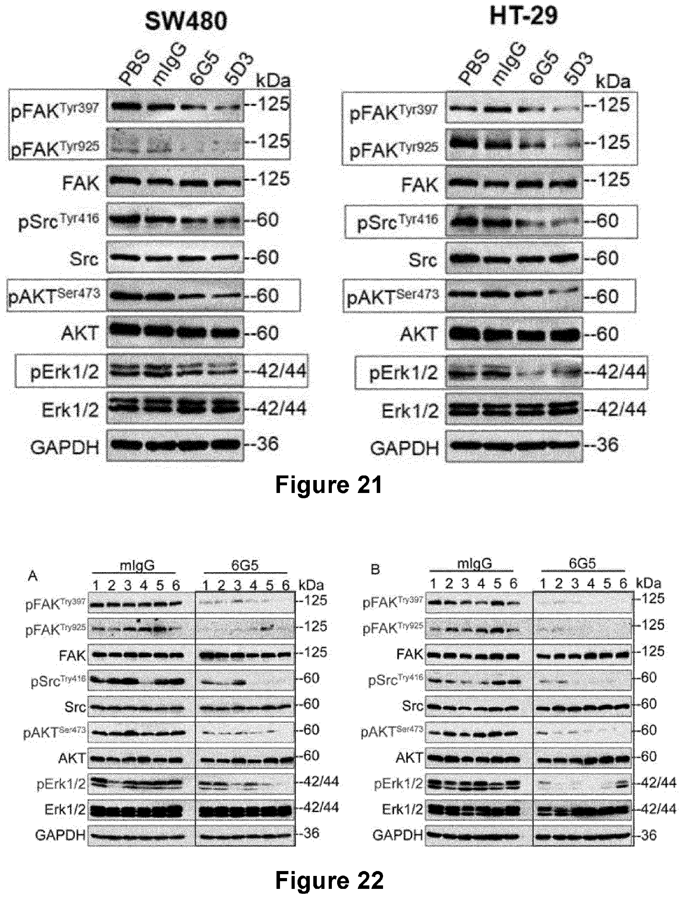

Control antibody mIgG (50 µg/ml) or the antibodies 5D3 and 6G5 were added to the supernatants of SW480 and HT-29 cell cultures. Cells were collected at 24 h, and the phosphorylation levels of major molecules involved in the Integrin-FAK signaling pathway were detected by Western blot, so as to detect the effect of the antibodies on activation of the Integrin-FAK pathway. The results were as shown in Figure 21, which showed that both 6G5 and 5D3 could reduce the phosphorylation levels of FAK, Src, Erk and Akt, which were major molecules downstream the Integrin signaling pathway, thereby inhibiting the activation of the Integrin-FAK signaling pathway.

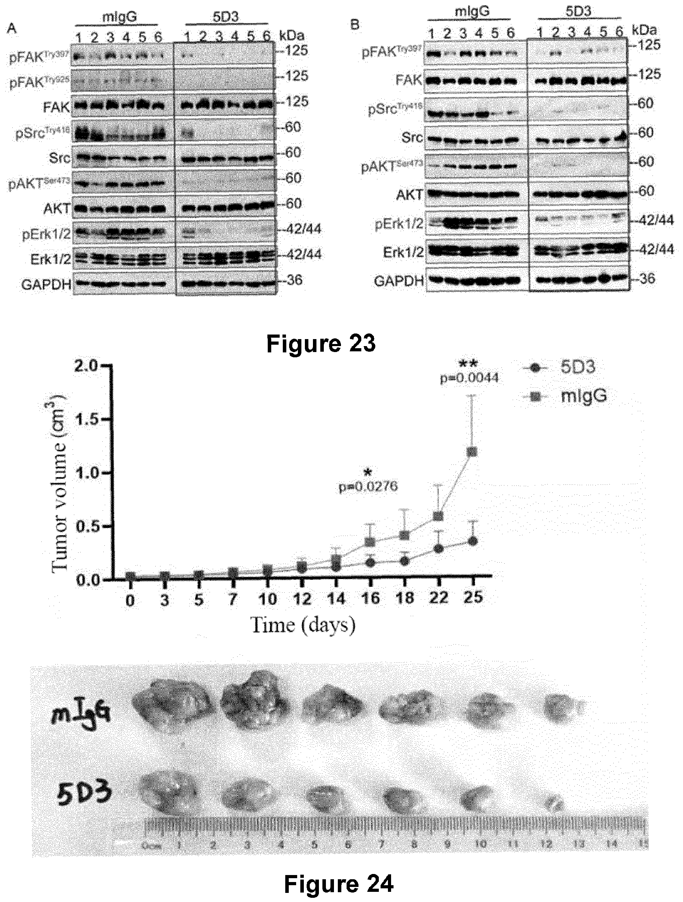

The procedure of *in vivo* experiment was the same as in Example 6, and the differences in the activation of Integrin-FAK signaling pathway between 6G5, 5D3 and the control group were compared at the end of the experiment. The results were as shown in Figures 22 and 23, which were basically consistent with the results detected *in vitro* at cell level.

### Example 8. Inhibition of the growth of osteosarcoma in vivo by monoclonal antibody 5D3

We next examined the effect of the monoclonal antibody 5D3 on the growth of osteosarcoma in nude mice *in vivo.*

The animal model was established by subcutaneously injecting a human osteosarcoma cell line 143B into nude mice (CDX), and after tumor formation, the animals were divided into two groups, with 6 animals in each group. One group of model was intravenously administered with the monoclonal antibody 5D3 at a dose of 5 mg/kg every 4 days, and the other group was treated with mouse IgG (mIgG) as a control, and the dosing scheme and dose were the same as those of 5D3. The tumor growth rate in the two groups of mice *in vivo* was dynamically observed, and the body weight changes in the mice were also dynamically monitored. At the end of the experiment, the differences in tumor volume and tumor weight between the 5D3-treated group and the control group were compared.

The experimental results were as shown in Figure 24 (5D3), and the tumor growth rate in mice of the experimental group was significantly slower than that of the control group. It can also be seen from the comparison of the volume of the dissected tumors that the overall tumor volume of the experimental group was significantly less than that of the mIgG control group.

The results showed that monoclonal antibody 5D3 had anti-tumor effect, and significantly inhibited the increase of tumor volume and weight compared with the control group.

### Example 9. Vκ4-1-IgLC polypeptide for use in assisting the diagnosis of multiple sclerosis

Multiple sclerosis (MS) is the most common type of central nervous system demyelinating disease. Multiple inflammatory demyelinating plaques are found in the white matter of the central nervous system at the acute active stage of the disease, and are more common in the optic nerve, spinal cord and brainstem. The disease is a common refractory disease in neurology. A high level of Igκ free Ig light chain is also found in serum and cerebrospinal fluid from patients with the disease, and is an important indicator to identify MS. However, the Igκ free Ig light chain has always been thought to be produced by B cells. We performed detection assays with antibodies capable of specifically recognizing the 28-amino-acid peptide (the amino acids at positions 5-32 of the full-length sequence, as shown in SEQ ID NO: 1) of Vκ4-1.

The serum from 10 patients with multiple sclerosis (MS) was selected, and the serum from 8 normal humans was used as a control. 1 µL of cryopreserved sample was taken and diluted with 10 µL of PBS, and subjected to denaturing SDS-PAGE electrophoresis. Then the protein was electrotransferred to a nitrocellulose membrane, and then detected by using a rabbit specific antibody obtained by immunization with the 28-amino-acid peptide (SEQ ID NO: 1) of Vκ4-1/Jκ.

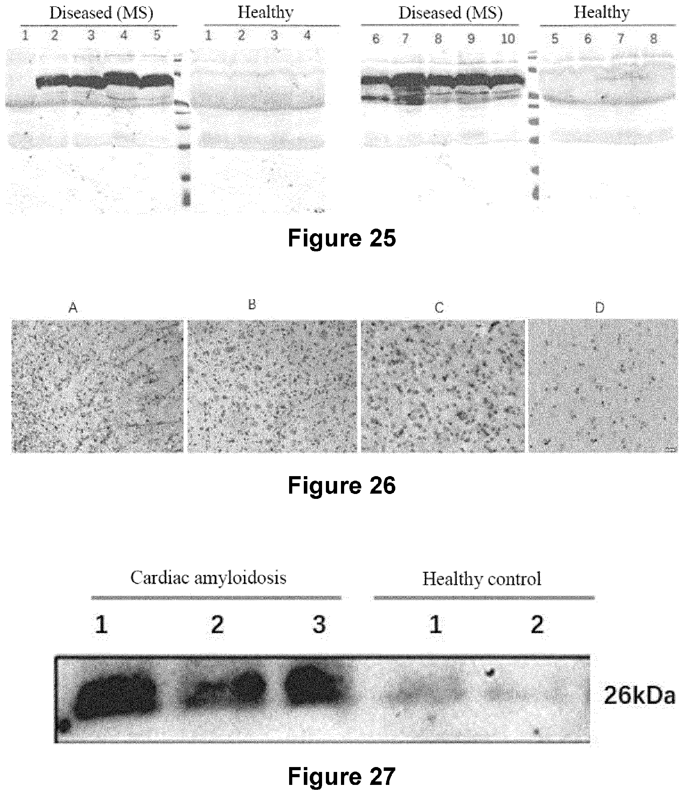

The results were as shown in Figure 25. The antibody specific for the 28-amino-acid peptide of Vκ4-1 specifically recognized the free Vκ4-1/Jκ light chain tetramer in the serum of MS patients, while the tetramer was not detected in normal humans (healthy controls). The results showed that there was a unique Vκ4-1 rearrangement in the Igκ free Ig light chain that occurred in the serum of MS patients, and the 28-amino-acid peptide could be used as an auxiliary diagnostic marker for MS.

### Example 10. Vx4-1-IgLC polypeptide for use in assisting the diagnosis of Alzheimer's disease

In our previous work, we found that Ig free light chains were widely present in non-B cells and are highly expressed in tumor cells, proliferative cells and inflammatory cells. It has been shown to be a cytoskeleton-associated protein, which could either be present in the cytoplasm to form filamentous structures, or could also be deposited in the extracellular matrix due to hydrophobic properties. Although there was no previous evidence suggesting that it was associated with amyloidosis in Alzheimer disease (AD), we made new discoveries by using 6G5 to stain the pathological sections from 10 patients with AD.

Pathological sections were taken from 10 patients with AD, routinely deparaffinized with xylene, and xylene was removed by gradient alcohol. After that, the sections were placed in boiling sodium citrate buffer for 5 minutes and naturally cooled. 1% Hydrogen peroxide was added to inhibit peroxidase (at room temperature for 10 minutes). The sections were washed with PBS and blocked with 10% horse serum (at room temperature for 10 minutes), and then incubated with 6G5 (at 4°C overnight). The sections were washed with PBS and stained with HRP-labeled anti-mouse IgG, and then observed under a microscope. The results were as shown in Figure 26.

The results showed that all 10 patients differently exhibited dendritic strong staining (Figure 26A) or focal and plaque (senile plaque) staining (Figure 26B) in the cerebral cortex. There were a small amount of light chain deposition at sites distal to the cortex, but the deposition was mainly in a diffusive state (Figure 26C), while there was no positive reaction in normal brain tissue (Figure 26D).

### Example 11. Vκ4-1-IgLC polypeptide for use in assisting the diagnosis of systemic amyloidosis

Systemic amyloidosis is a refractory disease characterized by irreversible organ failure caused by the deposition of free Ig light chains in a variety of tissues. This pathogenic free Ig light chain has long been thought to be produced by B cells. However, our previous work (including protein extraction, amino acid and nucleotide sequence alignment, etc.) found that the Ig free light chain with a Vκ4-1 variable region produced by non-B cells was highly homologous to the free Ig light chain that caused systemic amyloidosis. Subsequently, we performed detection experiments with 6G5, a monoclonal antibody specific for the 28-amino-acid peptide (positions 5-32) of Vκ4-1.

The serum from 3 patients with cardiac amyloidosis was selected, and the serum from 2 normal humans was used as a control. 1 µL of serum sample was taken and diluted with 10 µL of PBS, and subjected to denaturing SDS-PAGE electrophoresis. Then the protein was electrotransferred to a nitrocellulose membrane, and then detected by using 6G5. The results were as shown in Figure 27.

The results showed that the antibody specific for Vκ4-1 could specifically recognize the free Igκ light chain in the serum of patients with systemic amyloidosis, but not the Igκ of normal human, indicating that the 28-amino-acid target polypeptide could be used as an auxiliary diagnostic marker for patients with systemic amyloidosis.

### Example 12. Vx4-1-IgLC polypeptide for use in assisting the diagnosis of inflammatory nephropathy

Almost 90% of kidney diseases are related to pathogenic Ig, for example, IgA nephropathy is caused by the deposition of IgA in the mesangial area of the glomerulus, membranous nephropathy is caused by the deposition of IgG between the glomerular podocytes and basement membrane, and renal amyloidosis is caused by the deposition of free light chains in the glomerular basement membrane. However, it has long been believed in the field that these Igs are derived from B cells, while the inventors have found that these pathogenic Igs are mainly produced by local non-immune cells.

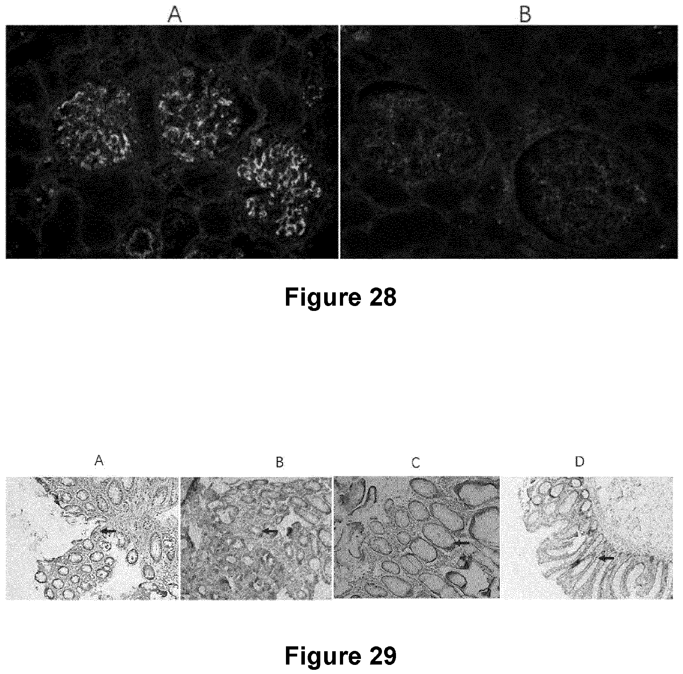

1% Hydrogen peroxide was added to frozen pathological sections taken from 5 cases of focal nephropathy to inhibit peroxidases (at room temperature for 10 minutes). The sections were washed with PBS and then blocked with 10% horse serum (at room temperature for 10 minutes). Then the sections were incubated with the monoclonal antibody 6G5 against the polypeptide having Vκ4-1 (at 4°C overnight), and mouse IgG was set as a control. The sections were washed with PBS and stained with FITC-labeled anti-mouse IgG, and then observed under a microscope. The results were as shown in Figure 28. The results showed that the glomeruli of all 5 patients showed strong fluorescence staining in basement membrane (A), no positive staining was observed for unrelated mice IgG (B), indicating that 6G5 could specifically recognize the deposition of Vκ4-1/Jκ-FLC in the glomerular basement membrane of patients with human focal nephropathy.

### Example 13. Vκ4-1-IgLC polypeptide for use in assisting the diagnosis of inflammatory gastrointestinal diseases

Ulcerative colitis and Crohn's disease are common refractory chronic inflammations in clinical practice, and often lead to inflammation-cancer transformation. Chronic gastritis also often turns into gastric cancer. The inventors found high levels of free Ig light chains (Vκ4-1/Jκ-Igκ) in the glandular epithelial cells of tissues of the above two diseases, which could promote the inflammatory process.

Pathological sections were taken from inflammatory colitis polyps, ulcerative colitis and chronic gastritis, routinely deparaffinized with xylene, and xylene was removed by gradient alcohol. The sections were placed in boiling sodium citrate buffer for 5 minutes and naturally cooled. 1% Hydrogen peroxide was added to inhibit peroxidase (at room temperature for 10 minutes). The sections were washed with PBS and blocked with 10% horse serum (at room temperature for 10 minutes), and then incubated with 5D3 (at 4°C overnight). The sections were washed with PBS and stained with HRP-labeled anti-mouse IgG, and then observed under a microscope. As can be seen from Figure 29, the results showed that inflammatory colitis polyps (A), ulcerative colitis (B), and chronic gastritis epithelial cells (C) were all positively stained (brown), while normal colon tissue (D) was negatively stained (blue).

The invention concept has been illustrated in details with reference to specific examples herein, and the above examples were illustrated only to help understand the central idea of the present invention. It should be noted that for those of ordinary skill in the art, any obvious modification, equivalent substitution or other improvement made without departing from the invention concept should be included in the protection scope of the present invention.

## Claims

1. A Vκ4-1-IgLC polypeptide, **characterized in that** the amino acid sequence is as shown in 5'-TQSPDSLVVSLGERASINCKSQRVSLG-3' (SEQ ID NO: 1).

2. Use of the polypeptide according to claim 1 in preparing a drug for diagnosing and/or treating malignant tumors or inflammatory diseases.

3. The use of claim 2, **characterized in that** the malignant tumors include at least one of the following: glioma, medulloblastoma, large cell lung cancer, esophageal cancer, gastric cancer, colorectal cancer, breast cancer, renal cell carcinoma, prostate cancer, liver cancer, pancreatic cancer, skin cancer, oral cancer, seminoma, osteosarcoma, leiomyosarcoma, angiosarcoma, liposarcoma, synovial sarcoma, rhabdomyosarcoma, B-cell lymphoma, T-cell lymphoma, leukemia and myeloma.

4. The use of claim 2, **characterized in that** the inflammatory diseases include at least one of the following: systemic lupus erythematosus, rheumatoid arthritis, multiple sclerosis, inflammatory bowel disease, inflammatory nephropathy, systemic amyloidosis, Alzheimer's disease and Parkinson's disease.

5. An antibody drug, **characterized in that** the polypeptide according to claim 1 is used as an antigen for preparing the antibody drug, and the antibody drug is a recognizing antibody or a blocking antibody.

6. The antibody drug according to claim 5, **characterized in that** it is a monoclonal antibody, and the amino acid sequence of its heavy chain variable region is as shown in SEQ ID NO: 3 or SEQ ID NO: 4.

7. The antibody drug according to claim 6, **characterized in that** the amino acid sequence of the light chain variable region is as shown in SEQ ID NO: 5.

8. A small molecule drug, **characterized in that** the gene sequence or mRNA sequence corresponding to the polypeptide according to claim 1 is used as a target for preparing the small molecule drug.

9. Use of the polypeptide according to claim 1 in preparing an inhibitor of the Integrin-FAK signaling pathway.
